# EUROPEAN PATENT SPECIFICATION

(11) **EP 0 745 381 B1**
(45) Date of publication and mention of the grant of the patent: **05.12.2001**
(21) Application number: 95670004.1
(22) Date of filing: 29.05.1995
(51) Int. Cl.: A61K 9/16, A61K 9/50, A61K 47/48

(54) **A process for preparing pharmaceutical compositions through deposition of complexes of drugs with cyclodextrins on inert or active cores**
Verfahren zur Herstellung von pharmazeutischen Zusammensetzungen durch die Absetzung von Arzenei/Zyklodextrin Komplexe auf inerte oder aktive Kerne
Procédé de préparation de compositions pharmaceutiques au moyen de la déposition de complexes comprenant des médicaments et des cyclodextrines sur des noyaux inertes ou actifs

(43) Date of publication of application: 04.12.1996
(73) Proprietor: TECNIMEDE-SOCIEDADE TECNICO-MEDICINAL, S.A., P-2685 Sacavém (PT)
(72) Inventor: De Sousa Goucha Jorge, Pedro Manuel, P-1500 Lisboa (PT)
(74) Representative: Pereira da Cruz, Joao

(56) References cited:
- EP-A- 0 241 806
- YAKUZAIGAKU, vol.53, no.4, 1993, JP pages 201 - 209 SUZUKI, YOSUKE ET AL. 'Bitterness-suppresive formulation of benexenate hydrochloride betadex prepared by hot melt granulation'

## Description

### Field of the Invention

The object of the present invention is a process for preparing pharmaceutical compositions comprising an inert or active core on which a complex consisting of a drug and a cyclodextrin is deposited.

### Background of the Invention

### 1. Cyclodextrins

Cyclodextrins are cyclic oligosaccharides containing a minimum of 6 D(+)-glucopyranose units connected by means of α-1,4 bonds. There are 3 different kinds of cyclodextrins, i.e., α,β and γ-cyclodextrins consisting of 6, 7 or 8 of such unities. They differ from each other in molecular weight, water solubility and in hollow diameter and are therefore able to form inclusion complexes with the most different kinds of compounds. There is a possibility of carrying out modifications in the cyclodextrin molecule with suitable substitutions. The solubility of modified cyclodextrins is generally higher and their capacity of forming inclusion complexes is also altered.

The external surface of the cyclodextrin molecule is hydrophilic and the inside of the cavity is hydrophobic. If a molecule adjusts itself, on the whole or in part, to the inside of the cavity, an inclusion complex is formed. In general, hydrophobic molecules have a greater affinity towards the cyclodextrin cavity. The complexes which are formed are stabilized by intramolecular forces, such as hydrophobic interaction forces, van der Waals forces and hydrogen bonds.

### 2. Obtaining inclusion complexes

Molecular inclusion or encapsulation may occur in the solid state, but while in solution, i.e., when the drug and the cyclodextrin are previously dissolved, the yield is higher. The methods which are more often used are the following:
- In solution:
   - Precipitation
   - Freeze drying
   - Spray Drying
   - Neutralization
- Others:
   - Kneading
   - Mixing
   - Mixing and Grinding
   - Sealed heating

Up to now, methods which comprise obtaining a solution of drug and cyclodextrin and which are the most valuable ones because they allow a greater interaction and a higher degree of compexation, have not proved to be easily applied at a pharmaceutical industry level, for two particular reasons:
- The evaporation of the solvents which are used in view of isolating the solid complex, requires the use of usually non-available equipment, a fact which is especially apparent as far as solid form production units are concerned (this being the case of lyophilizators and spray-dryers);
- Using methods comprising the previous solubilization of the components (drug and cyclodextrin), the solid complex is isolated in the form of a microcrystalline or amorphous powder, the size and shape of which render the formulation difficult and require a subsequent processing (generally granulation, so as to confer density, flowability, uniformity and adherence) in view of obtaining a final administration form (sachet, tablet or capsule).

The main advantages of the process which is now described, as compared to former methods, are the following:
- It resorts merely to equipment which is usually available in the pharmaceutical industry and, more precisely, to production units of solid pharmaceutical forms, such as those which enable to carry out the coating in a pan or fluid bed;
- The solid drug/cyclodextrin complex deposited on a matrix such as the one obtained by means of this process, in a single operation, is easily incorporated in a pharmaceutical composition;
- The release profile of the complex of the pharmaceutical composition may be easily manipulated, by simultaneously employing formulation adjuvants, such as polymeric substances usually used by the pharmaceutical industry for that aim.

### 3. Coating

Coating is a well known method used by the pharmaceutical industry consisting generally in covering granules or tablets with polymeric substances having no therapeutic activity (excipients). The main aims of the coating process are:
- improving the aspect of the pharmaceutical preparation;
- protecting the product from the influence of light, moisture or oxygen;
- preventing any disagreeable odour or flavour;
- changing the release rate of the drug from the pharmaceutical composition in order to:
   - prevent the degradation of the pharmaceutical preparation by gastric juice;
   - extend the absorption thereof.

In general, the substances which form the coating are applied on the cores in the form of solutions or suspensions, and for that reason the process requires the simultaneous evaporation of the solvents used therein. In some less frequent cases, the same substances are applied in the form of powder, the adherence to the cores being guaranteed by moistening with solutions or suspensions comprising agglutinating agents. In this case, the simultaneous evaporation of the solvents is also required.

The coating with pharmaceuticals is less frequent, and is mainly used in the following instances:
- to prevent the incompatibility between two drugs in the same formulation (one of them being dispersed in the core and the other in the coating layer);
- preparing modified release formulations by first covering the inert granules with the drug and then coating them with a solution or a suspension of the polymeric agent.

In each case the drug is generally applied in the form of a powder.

### Summary of the Invention

The process for preparing complexes of drugs with cyclodextrins through deposition, which is the object of the present invention, contrary to the known processes for obtaining complexes by first dissolving the drug and the cyclodextrin (precipitation, lyophilization or spray drying), has the advantage of providing administration forms the composition of which contains drugs and cyclodextrins, by means of an easy process, without it being necessary to resort to methods such as filtration, lyophilization, spray drying, granulation and eventually compactation and compression. This is due to the possibility of simultaneously carrying out the evaporation of the solvent and the deposition of the complex on a core , a process which confers to the resulting product suitable density, flowability and uniformity characteristics and, in the case of subsequent compression, good adherence. The simultaneous use of adequate adjuvants, such as substances of a polymeric nature, also enables one to obtain the desired release profile for the drug in question. The process which is the object of the present invention has thus an easy industrial application.

### Detailed Description of the Invention.

The object of the present invention is a process for preparing pharmaceutical compositions comprising an inert or active core on which a complex consisting of a drug and cyclodextrin is deposited.

Said process essentially comprises the following steps:
A) Preparing a liquid phase consisting essentially of a natural or modified cyclodextrin solution in an appropriate solvent or solvent mixture;
B) Dissolving a certain amount of drug in the solution obtained in A); and
C) Spraying the solution obtained in B) on inert or active cores, which may be in the form of powder, granules or small tablets, with the simultaneous elimination of the solvent by evaporation, in order to achieve the deposition of the complex on said inert or active cores.

As a variant of this process the spraying of the solution in step C) may be followed by the simultaneous addition of a polymer in the form of a powder.

Another alternative process which may be used in the preparation of said pharmaceutical compositions consists essentially in the following steps:
A) Preparing a liquid phase essentially consisting of a natural or modified cyclodextrin solution in a solvent or in an appropriate solvent mixture;
B) Dissolving a certain amount of the drug in the solution obtained in A);
C) Preparing a second liquid phase essentially consisting of a solution or suspension of a polymer in the same solvent or solvent mixture used in A), or in a solvent capable of being mixed with the latter;
D) Adding to one another, with stirring, the liquid phases obtained in B) and C) in order to immediately obtain a solution or suspension of the drug/cyclodextrin complex within the polymer; and
E) Spraying the solution or suspension obtained in D) on the inert or active cores, which may be in the form of powder, granules or small tablets, and simultaneously eliminating the solvent by evaporation, in order to achieve the deposition of the complex on said inert or active cores.

In any of the above mentioned processes the solvent used may be water or an aqueous mixture, or it may be organic or a mixture of organic solvents. It is especially preferred to use as solvent water or an aqueous mixture .

Dissolution of the drug in step B) may be adjuvated with various methods which resort, in particular, to tensides, to altering the pH of the solution obtained in A), rising the temperature of said solution, etc.

It is convenient that the drug/cyclodextrin complex be totally dissolved in the solution or suspension to be sprayed on the inert or active cores.

The polymer used in the second step and in the variant of the first step should allow the instant release of the drug/cyclodextrin complex or enable its release to be modified after contacting the fluids of the gastrointestinal tract.

It may be convenient, in the processes referred to in the previous paragraph, to carry out the dissolution of a plastifying agent in the solution or suspension to be sprayed on the inert or active cores.

The drug which is present in the drug/cyclodextrin complex is usually different from the drug present in the active cores. The drug which is present in the active cores is preferably in the form of a drug/cyclodextrin complex.

The cyclodextrin used in step A) is preferably a dimethyl-β-cyclodextrin. One of the favourite drugs to be used in step B) is nitrendipine. Although other polymers may be used in step C) of the second process and in the variant of the first process, hydroxypropylmethylcellulose is preferred. It is particularly preferred that in any of the processes the complex deposited is the nitrendipine/dimethyl-β-cyclodextrin complex (1:2).

The preparation of the solution containing the complex and the eventual addition of same to the solution or suspension of the polymer is carried out with the aid of stirring devices generally used in the pharmaceutical industry for preparing solutions or suspensions.

The deposition of the complex on inert or active cores is carried out by means of known coating methods used in the pharmaceutical industry, such as pan coating or coating on a fluid bed (top-spray, bottom spray Wurster or tangencial spray). In order to obtain the final administration form, the product may be processed by one of the known methods generally used in the pharmaceutical industry such as suspension, compression or filling in hard gelatin capsules or sachets.

### Brief Description of the Drawings

Figure 1 shows the thermogram obtained by differential scanning calorimetry (DSC) concerning granules of an instant release oral composition, obtained by deposition of the nitrendipine/dimethyl-β-cyclodextrin complex (1:2) on inert granules.

Figure 2 shows the thermogram obtained by differential scanning calorimetry DSC) concerning granules of an extended release oral composition, obtained by deposition of the nitrendipine/dimethyl-β-cyclodextrin complex (1:2) on inert granules.

In both cases the endothermic peak disappears around 160°C, due to the melting of nitrendipine, which suggests that nitrendipine forms an inclusion complex with dimethyl-β-cyclodextrin. In the compositions obtained by deposition, the endothermic peak around 190°C is due to the melting of the inert granules' components.

The present invention is further illustrated by means of the following non-limiting Examples.

### Example 1

### Preparation of an instant release oral pharmaceutical composition by deposition of the nitrendipine/dimethyl-β-cyclodextrin complex (1:2) on inert granules.

| | | |
|---|---|---|
| **Phase 1** | Nitrendipine | 2 500 mg |
| | Dimethyl-β-cyclodextrin | 18 176 mg |
| | Ethanol | 50 ml |
| | Dichloromethane | 50 ml |
| **Phase 2** | ---------------- | ------------ |

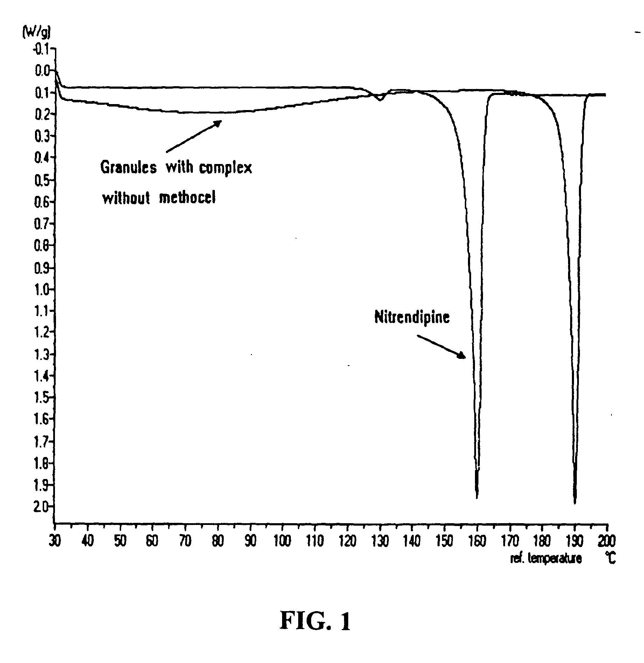

Phase 1 was sprayed, using the fluid bed coating method (top-spray), on inert granules consisting of 25% starch and 75% saccharose with a diameter ranging from 500 to 590 µm. The thermogram obtained by differential scanning calorimetry (DSC) of the resulting granules containing the complex shows no endothermic peak around the melting temperature of nitrendipine (Fig. 1).

### Example 2

### Preparation of an extended release oral pharmaceutical composition by deposition of the nitrendipine/dimethyl-β-cyclodextrin complex (1:2) on inert granules

| | | |
|---|---|---|
| **Phase 1** | Nitrendipine | 4 352.6 mg |
| | Dimethyl-β-cyclodextrin | 31 644.8 mg |
| | Ethanol | 100 ml |
| | Dichloromethane | 100 ml |
| **Phase 2** | Hydroxypropylmethylcellulose (Methocel K100M) | 4 000.0 mg |
| | Ethanol | 150 ml |
| | Dichloromethane | 150 ml |

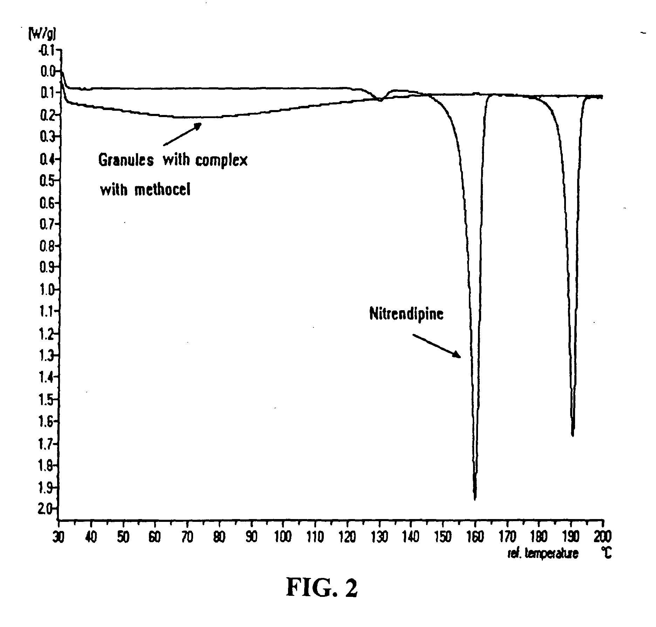

Phase 1 was added with stirring to Phase 2. The resulting solution was sprayed using the fluid bed coating method (top spray) on inert granules consisting of 25% starch and 75% saccharose with a diameter ranging from 500 to 590 µm. The thermogram obtained by differential scanning calorimetry (DSC) of the resulting granules containing the complex shows no endothermic peak around the melting temperature of nitrendipine (Fig. 2).

The dissolution characteristics of the granules obtained according to the two Examples and containing equal amounts of nitrendipine (20 mg) were compared at 37°C in 1000 ml water, with the addition of 0,5% of Tween 80, using apparatus 2 of USP XXIII (paddle method). The results (Table 1) show that nitrendipine dissolves more slowly in the second case due to the simultaneous deposition of the complex and of the polymeric agent

**Table 1**

| Nitrendipine concentrations (µg/ml) | | |
|---|---|---|
| **Time (min)** | **Example 1** | **Example 2** |
| 1 | 10.7 | |
| 2 | 11.3 | 4.5 |
| 5 | 11.7 | 5.6 |
| 10 | | 9.9 |
| 15 | | 13.8 |
| 20 | | 15.9 |
| 30 | | 17.6 |
| 40 | | 17.9 |
| 45 | | 18.0 |

## Claims

1. A process for preparing pharmaceutical compositions through deposition of complexes of drugs with cyclodextrins on inert or active cores, **characterized in that** it comprises the following steps:
A) Preparing a liquid phase essentially consisting of a natural or modified cyclodextrin solution in an appropriate solvent or solvent mixture;
B) Dissolving a certain amount of drug in the solution obtained in A); and
C) Spraying the solution obtained in B), accompanied or not by simultaneous addition of a polymer in powder form, on inert or active cores, which may be in the form of powder, granules or small tablets, and simultaneously eliminating the solvent by evaporation in order to achieve the deposition of the complex on same inert or active cores.

2. A process for preparing pharmaceutical compositions through deposition of drug/cyclodextrin complexes on inert or active cores, **characterized in that** it comprises the following steps:
A) Preparing a liquid phase essentially consisting of a natural or modified cyclodextrin solution in a solvent or an appropriate solvent mixture;
B) Dissolving a certain amount of the drug in the solution obtained in A);
C) Preparing a second liquid phase essentially consisting of a solution or suspension of a polymer in the same solvent or solvent mixture used in A), or in a solvent miscible with the latter;
D) Adding to one another, with stirring, the liquid phases obtained in B) and C), in order to immediately obtain a solution or suspension of the drug/cyclodextrin complex dispersed within the polymer; and
E) Spraying the solution or suspension obtained in D) on inert or active cores which may be in the form of powder, granules or small tablets, and simultaneously eliminating the solvent by evaporation, in order to obtain the deposition of the complex on said inert or active cores.

3. A process according to Claim 1 or 2, **characterized in that** the solvent used is water or an aqueous mixture.

4. A process according to Claim 1 or 2, **characterized in that** the solvent used is organic or a mixture of organic solvents.

5. A process according to Claim 1 or 2, **characterized in that** the dissolution of the drug in step B) is adjuvated by means of one or more tensides.

6. A process according to Claim 1 or 2, **characterized in that** the dissolution of the drug in step B) is adjuvated by modifying the pH of the cyclodextrin solution obtained in A).

7. A process according to Claim 1 or 2, **characterized in that** the dissolution of the drug in step B) is adjuvated by rising the temperature of the cyclodextrin solution obtained in A).

8. A process according to Claim 1 or 2 , **characterized in that** the drug/cyclodextrin complex is not totally dissolved in the suspension to be sprayed on the inert or active cores.

9. A process according to Claim 1 or 2, **characterized in that** the polymer used enables the modification of the "in vitro" dissolution rate of the drug from the coated cores.

10. A process according to Claim 1 or 2, **characterized in that** it comprises dissolving a plastifying agent in the solution or suspension to be sprayed on inert or active cores.

11. A process according to Claim 1 or 2, **characterized in that** the drug present in the drug/cyclodextrin complex which is deposited is different from the drug present in the active cores.

12. A process according to Claim 1 or 2, **characterized in that** the drug present in the active cores is in the form of a drug/cyclodextrin complex.

13. A process according to Claim 1 or 2, **characterized in that** the cyclodextrin used in step A) is dimethyl-β- cylodextrin.

14. A process according to Claim 1 or 2, chracterized in that the drug used in step B) is nitrendipine.

15. A process accordins to Claim 1 or 2, **characterized in that** the polymer used in step C) is hydroxypropylmethylcellulose.

16. A process according to Claim 1 or 2, **characterized in that** the solvent used is ethanol, dichloromethane or a mixture thereof.

17. A process according to Claim 1 or 2, **characterized in that** the complex deposited is the nitrendipine/dimethyl-β-cyclodextrin complex (1:2).

## Patentansprüche

1. Verfahren zur Herstellung pharmazeutischer Zubereitungen durch Ablagerung von Cyclodextrin enthaltende Arzneimittelkomplexe auf inerte oder aktive Kerne, **dadurch gekennzeichnet dass** es die folgenden Stufen umfasst:
A) Vorbereitung einer flüssigen Phase bestehend aus einer natürlichen oder modifizierten Cyclodextrinlösung in einem geeigneten Lösungsmittel oder Gemisch von Lösungsmitteln;
B) Auflösen einer bestimmten Menge des Arzneimittels in der in A) erhaltenen Lösung; und
C) Sprühen der in B) erhaltenen Lösung begleitet oder nicht von der gleichzeitigen Zugabe eines in puderform vorliegendes Polymer auf inerte oder aktive Kerne, die in der Form von Pulvern, Granulate oder kleine Tabletten vorliegen können, und gleichzeitige Entfernung des Lösungsmittel durch Eindampfen um die Ablagerung des Komplexes auf den erwähnten inaktiven oder aktiven Kerne zu veranlassen.

2. Verfahren zur Herstellung pharmazeutischer Zubereitungen durch Ablagerung von Cyclodextrin enthaltende Arzneimittelkomplexe auf inerte oder aktive Kerne, **dadurch gekennzeichnet dass** es die folgenden Stufen umfasst:
A) Vorbereitung einer flüssigen Phase bestehend aus einer natürlichen oder modifizierten Cyclodextrinlösung in einem geeigneten Lösungsmittel oder Gemisch von Lösungsmitteln;
B) Auflösen einer bestimmten Menge des Arzneimittels in der in A) erhaltenen Lösung;
C) Vorbereitung einer zweiten flüssigen Phase, wesentlich aus einer Polymerlösung oder Suspension bestehend, im selben in A) benutzten Lösungsmittel oder Gemisch von Lösungsmitteln, oder in einem mit diesen mischbaren Lösungsmittel;
D) Zugabe unter Rühren der beiden in B) und C) erhaltenen flüssigen Phasen zur sofortigen Erhaltung einer Lösung oder Suspension des innerhalb des Polymers dispergiertes Arzneimittel/Cyclodextrin Komplexes; und
E) Sprühen der in D) erhaltenen Lösung oder Suspension auf inerte oder aktive Kerne die in Form von Pulvern, Granulate oder kleine Tabletten vorliegen können, und gleichzeitige Entfernung des Lösungsmittel durch Eindampfen um die Ablagerung des Komplexes auf den erwähnten inerte oder aktive Kerne zu bewirken;

3. Verfahren gemäss Anspruch 1 oder 2, **dadurch gekennzeichnet dass** das verwendete Lösungsmittel Wasser oder ein wässriges Gemisch ist.

4. Verfahren gemäss Anspruch 1 oder 2, **dadurch gekennzeichnet dass** das verwendete Lösungsmittel organisch oder ein Gemisch organischer Lösungsmittel ist.

5. Verfahren gemäss Anspruch 1 oder 2, **dadurch gekennzeichnet dass** die Auflösung des Arzneimittels in Stufe B) mit der Hilfe eines oder mehrerer Tenside durchgeführt wird.

6. Verfahren gemäss Anspruch 1 oder 2, **dadurch gekennzeichnet dass** die Auflösung des Arzneimittels in Stufe B) durch Modifizierung des pH der in A) erhaltenen Cyclodextrinlösung veranlasst wird.

7. Verfahren gemäss Anspruch 1 oder 2, **dadurch gekennzeichnet dass** die Auflösung des Arzneimittels in Stufe B) durch Erhöhung der Temperatur der in A) erhaltenen Cyclodextrinlösung veranlasst wird.

8. Verfahren gemäss Anspruch 1 oder 2, **dadurch gekennzeichnet dass** das Arzneimittel/Cyclodextrin Komplex nicht völlig in der auf den inerten oder aktiven Kerne zu sprühender Suspension gelöst ist.

9. Verfahren gemäss Anspruch 1 oder 2, **dadurch gekennzeichnet dass** das eingesetzte Polymer die Änderung des "in vitro" Lösungsanteil des Arzneimittels der beschichteten Kerne ermöglicht

10. Verfahren gemäss Anspruch 1 oder 2, **dadurch gekennzeichnet dass** es die Auflösung eines plastifizierendes Mittels in der auf den inerten oder aktiven Kerne zu sprühender Lösung oder Suspension unfasst.

11. Verfahren gemäss Anspruch 1 oder 2, **dadurch gekennzeichnet dass** das im Arzneimittel/Cyclodextrin Komplex vorhandene, abgelagerte Arzneimittel verschieden von dem in den aktiven Kerne vorhandene Arzneimittel ist.

12. Verfahren gemäss Anspruch 1 oder 2, **dadurch gekennzeichnet dass** das in den aktiven Kerne vorhandene Arzneimittel in Form eines Arzneimittel/Cyclodextrin Komplex vorkommt

13. Verfahren gemäss Anspruch 1 oder 2, **dadurch gekennzeichnet dass** die in Stufe A) verwendete Cyclodextrin die Dimethyl-β-Cyclodextrin ist.

14. Verfahren gemäss Anspruch 1 oder 2, **dadurch gekennzeichnet dass** das in Stufe B) verwendete Arzneimittel Nitrendipin ist.

15. Verfahren gemäss Anspruch 1 oder 2, **dadurch gekennzeichnet dass** das in Stufe C) verwendete Polymer Hydroxypropylmethylzellulose ist.

16. Verfahren gemäss Anspruch 1 oder 2, **dadurch gekennzeichnet dass** das verwendete Lösungsmittel Ethanol, Dichloromethan oder ein Gemisch derselben ist.

17. Verfahren gemäss Anspruch 1 oder 2, **dadurch gekennzeichnet dass** das abgelagerte Komplex das Nittrodipin/Dimethyl-β-Cyclodextrin Komplex (1:2) ist.

## Revendications

1. Procédé pour la préparation de compositions pharmaceutiques à travers le dépôt de complexes de produits pharmaceutiques avec des cyclodextrines sur des noyaux inertes ou actifs, **caractérisé en ce qu'**il comprend les étapes suivantes:
A) Préparation d'une phase liquide constituée essentiellement d'une solution de cyclodextrine naturelle ou modifiée dans un solvant ou mélange de solvants appropriés;
B) Dissolution d'une quantité déterminée de produit pharmaceutique dans la solution obtenue lors de l'étape A); et
C) Pulvérisation de la solution obtenue lors de l'étape B), accompagnée ou pas de l'addition simultanée d'un polymère sous forme de poudre, sur des noyaux inertes ou actifs, qui peuvent être des poudres, des granules ou de petits comprimés, avec l'élimination simultanée du solvant par évaporation afin d'obtenir le dépôt du complexe sur les mêmes noyaux inertes ou actifs.

2. Procédé pour la préparation de compositions pharmaceutiques à travers le dépôt de complexes de produits pharmaceutiques/cyclodextrines sur des noyaux inertes ou actifs, **caractérisé en ce qu'**il comprend les étapes suivantes:
A) Préparation d'une phase liquide constituée essentiellement d'une solution de cyclodextrine naturelle ou modifiée dans un solvant ou mélange de solvants appropriés;
B) Dissolution d'une quantité déterminée du produit pharmaceutique dans la solution obtenue lors de l'étape A); et
C) Préparation d'une deuxième phase liquide constituée essentiellement d'une solution ou d'une suspension d'un polymère dans le même solvant ou mélange de solvants utilisés lors de l'étape A), ou dans un solvant miscible à ces derniers;
D) Addition, sous agitation, des phases liquides obtenues lors des étapes B) et D), afin d'obtenir immédiatement une solution ou suspension du complexe de produit pharmaceutique/cyclodextrine dispersé à l'intérieur du polymère; et
E) Pulvérisation de la solution obtenue lors de l'étape D) sur des noyaux inertes ou actifs, qui peuvent être des poudres, des granules ou de petits comprimés, avec l'élimination simultanée du solvant par évaporation afin d'obtenir le dépôt du complexe sur les mêmes noyaux inertes ou actifs.

3. Procédé selon la Revendication 1 ou 2, **caractérisé en ce que** le solvant utilisé est l'eau ou un mélange aqueux.

4. Procédé selon la Revendication 1 ou 2, **caractérisé en ce que** le solvant utilisé est organique ou un mélange de solvants organiques.

5. Procédé selon la Revendication 1 ou 2, **caractérisé en ce que** la dissolution du produit pharmaceutique lors de l'étape B) est adjuvée au moyen d'un ou plusieurs tensioactifs.

6. Procédé selon la Revendication 1 ou 2, **caractérisé en ce que** la dissolution du produit pharmaceutique lors de l'étape B) est adjuvée au moyen de la modification du pH de la solution de cyclodextrine obtenue lors de l'étape A).

7. Procédé selon la Revendication 1 ou 2, **caractérisé en ce que** la dissolution du produit pharmaceutique lors de l'étape B) est adjuvée au moyen de l'élévation de la température de la solution de cyclodextrine obtenue lors de l'étape A).

8. Procédé selon la Revendication 1 ou 2, **caractérisé en ce que** le complexe de produit pharmaceutique/cyclodextrine n'est pas totalement dissous dans la suspension à être pulvérisée sur les noyaux inertes ou actifs.

9. Procédé selon la Revendication 1 ou 2, **caractérisé en ce que** le polymère utilisé permet la modification du taux de dissolution "in vitro" du produit pharmaceutique des noyaux revêtus.

10. Procédé selon la Revendication 1 ou 2, **caractérisé en ce qu'**il comprend la dissolution d'un agent plastifiant dans la solution ou suspension à être pulvérisée sur les noyaux inertes ou actifs.

11. Procédé selon la Revendication 1 ou 2, **caractérisé en ce que** le produit pharmaceutique présent dans le complexe de produit pharmaceutique/cyclodextrine qui est déposé est différent du produit pharmaceutique présent dans les noyaux inertes.

12. Procédé selon la Revendication 1 ou 2, **caractérisé en ce que** le produit pharmaceutique présent dans les noyaux actifs est sous forme d'un complexe de produit pharmaceutique/cyclodextrine.

13. Procédé selon la Revendication 1 ou 2, **caractérisé en ce que** la cyclodextrine utilisée lors l'étape A) est la diméthyle-β-cyclodextrine.

14. Procédé selon la Revendication 1 ou 2, **caractérisé en ce que** le produit pharmaceutique utilisé lors l'étape B) est la nitrendipine.

15. Procédé selon la Revendication 1 ou 2, **caractérisé en ce que** le polymère utilisé lors l'étape C) est l'hydroxypropylméthylcellulose.

16. Procédé selon la Revendication 1 ou 2, **caractérisé en ce que** le solvant utilisé est l'éthanol, la dichlorométhane ou un mélange des deux.

17. Procédé selon la Revendication 1 ou 2, **caractérisé en ce que** le complexe déposé est le complexe nitrendipine/diméthyle-β-cyclodextrine (1:2).
